# EUROPÄISCHE PATENTSCHRIFT

(11) **EP 1 349 535 B1**
(45) Veröffentlichungstag und Bekanntmachung des Hinweises auf die Patenterteilung: **02.11.2006**
(21) Anmeldenummer: 02716059.7
(22) Anmeldetag: 03.01.2002
(51) Int. Cl.: A61Q 5/10, A61K 8/49, C09B 69/00

(54) **INDOL-/INDOLIN-HYBRIDFARBSTOFFE UND -HYBRIDFARBSTOFFVORPRODUKTE**
INDOLE/INDOLINE BASED HYBRID DYES AND INDOLE/INDOLINE BASED HYBRID DYE INTERMEDIATE PRODUCTS
COLORANTS HYBRIDES A BASE D'INDOLE ET D'INDOLINE ET PRODUITS DE BASE DE COLORANTS HYBRIDES A BASE D'INDOLE ET D'INDOLINE

(30) Priorität: 10.01.2001 DE 10100938
(43) Veröffentlichungstag der Anmeldung: 08.10.2003
(73) Patentinhaber: Henkel Kommanditgesellschaft auf Aktien, 40589 Düsseldorf (DE)
(72) Erfinder: HOLLENBERG, Detlef, 40699 Erkrath (DE); NAUMANN, Frank, 40593 Düsseldorf (DE); HÖFFKES, Horst, 40595 Düsseldorf (DE); ROSE, David, 40723 Hilden (DE)
(86) Internationale Anmeldenummer: PCT/EP2002/000013
(87) Internationale Veröffentlichungsnummer: WO 2002/055609

(56) Entgegenhaltungen:
- WO-A-99/20234
- DE-A- 2 716 671
- US-A- 4 961 754
- US-A- 5 785 717

## Beschreibung

Die Erfmdung betrifft neue Indol-/Indolin-Hybridfarbstoffe und -hybridfarbstoffvorprodukte, die insbesondere zum Färben von Keratinfasern geeignet sind, die Verwendung dieser Farbstoffe und Farbstoffvorprodukte sowie diese Farbstoffe und/oder Farbstoffvorprodukte enthaltende Färbemittel.

Im Rahmen der Produkte, die zur kosmetischen Behandlung des menschlichen Körpers bereitgestellt werden, spielen Mittel zur Veränderung oder Nuancierung der Farbe des Kopfhaares eine herausragende Rolle. Sieht man von den Blondiermitteln, die eine oxidative Aufhellung der Haare durch Abbau der natürlichen Haarfarbstoffe bewirken, ab, so sind im Bereich der Haarfärbung im wesentlichen drei Typen von Haarfärbemitteln von Bedeutung:

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe mehr benötigen. Zu diesen Farbstoffen gehört beispielsweise das bereits aus dem Altertum zur Färbung von Körper und Haaren bekannte Henna.

Weiterhin hat in jüngster Zeit ein neuartiges Färbeverfahren große Beachtung gefunden. Bei diesem Verfahren werden Vorstufen des natürlichen Haarfarbstoffes Melanin, insbesondere Derivate des Indols oder Indolins, auf das Haar aufgebracht und bilden im Rahmen oxidativer Prozesse im Haar praktisch naturidentische Farbstoffe aus. Ein solches Verfahren mit 5,6-Dihydroxyindolinen als Farbstoffvorprodukte wurde in der EP-B1-530 229 beschrieben. Bei, insbesondere mehrfacher, Anwendung von Mitteln mit 5,6-Dihydroxyindolin ist es möglich, Personen mit ergrauten Haaren die natürliche Haarfarbe wiederzugeben. Die Ausfärbung kann dabei mit Luftsauerstoff als einzigem Oxidationsmittel erfolgen, so daß auf keine weiteren Oxidationsmittel zurückgegriffen werden muß.

Zwar ist es prinzipiell möglich, Färbemittel mit lediglich einem Farbstoff oder einer Farbstoffvorstufe zu formulieren. Mit Ausnahme weniger Produkte, die z. B. Vorläufer des Melanins enthalten, haben solche Färbemittel in der Praxis aber nur geringe Bedeutung erlangt.

Kommerzielle Haarfärbeprodukte enthalten vielmehr üblicherweise eine Mischung von etwa 3 bis 8 unterschiedlichen Farbstoffen und/oder Farbstoffvorprodukten. Die einzelnen Farbstoffe weisen aber in der Regel unterschiedliche Aufziehvermögen, Licht-, Schweiß-, Reib- und Waschechtheiten auf, die zudem noch stark von den strukturellen Eigenschaften und dem Pflegezustand des Haares abhängig sein können. Diese Unterschiede sind vor allem dann ausgeprägt, wenn, wie für viele Nuancen bisher unverzichtbar, direktziehende Farbstoffe zur Einstellung der Nuance in Oxidationsfärbemitteln eingesetzt werden.

Es bedarf daher bei der Entwicklung neuer Haarfärbemittel häufig umfangreicher Versuche, nicht nur um bestimmte Nuancen zu erzielen, sondern vor allem um sicherzustellen, daß die Färbung über den gewünschten Zeitraum sowohl in der Nuance als auch in der Intensität stabil ist.

Es wurde nunmehr überraschenderweise gefunden, daß viele der oben genannten Probleme ganz oder zumindest teilweise vermieden werden können, wenn Substanzen eingesetzt werden, die sowohl über die Eigenschaften eines Vorläufers des Melanins als auch über die Eigenschaften eines Oxidationsfarbstoffvorproduktes verfügen. Insbesondere hat sich gezeigt, daß die Farbstoffe über ein sehr gutes, mit bekannten Haarfarbstoffen bzw. Haarfarbstoffvorprodukten vergleichbares Aufziehvermögen auf das Haar verfügen und zu brillanten, intensiven Haarfärbungen führen. Aufgrund der molekularen Verknüpfung läßt sich so das Problem unterschiedlicher Echtheitseigenschaften der Farbstoffe bzw. Farbstoffvorprodukte in vielen Fällen weitgehend überwinden.

Solche in Haarfärbemitteln einsetzbare Substanzen, die im weiteren als "Hybridfarbstoffe" bezeichnet werden, sind neu.

Ein erster Gegenstand der vorliegenden Erfindung sind daher Indol-/Indolin-Hybridfarbstoffe und -hybridfarbstoffvorprodukte, insbesondere zum Färben keratinischer Fasern, der Struktur (I),

X - S - Y (I)

in der
X steht für eine Gruppe, die abgeleitet ist von einem Derivat des Indols oder Indolins als Vorläufer des Melanins;
Y steht für eine Gruppe, die abgeleitet ist von
- einem Oxidationsfarbstoffvorprodukt vom Kuppler- oder Entwicklertyp
S steht für ein Strukturelement, das ein gemeinsamer Bestandteil der Gruppen X und Y ist,
eine direkte Bindung oder wenigstens eine Spacer-Gruppe.

Die Verbindungen gemäß Struktur (I) sind mit üblichen Syntheseverfahren der organischen Chemie zugänglich. In diesem Zusammenhang wird ausdrücklich auf die unten genannten Synthesebeispiele verwiesen.

Wie bereits oben ausgeführt, bilden die Strukturprinzipien bekannter Farbstoffklassen die Basis für die neu entwickelten Hybridfarbstoffe.

Die Gruppe X leitet sich ab von einem Vorläufer des Melanins, ausgewählt aus den Derivaten des Indols und Indolins. Im Rahmen der vorliegenden Erfindung werden unter "Vorläufer des Melanins" solche Derivate des Indols und des Indolins verstanden, die im Rahmen eines oxidativen Prozesses in der Lage sind, Melaninfarbstoffe oder entsprechende Melaninfarbstoff-Derivate auszubilden.

Erfindungsgemäß bevorzugt leitet sich die Gruppe X im Rahmen dieser Ausführungsform von Indolen und Indolinen ab, die wenigstens eine Hydroxy- und/oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. Indole und Indoline mit zwei dieser Gruppen, insbesondere zwei Hydroxygruppen, von denen eine oder beide verethert oder verestert sein können, sind besonders bevorzugt.

Erfindungsgemäß besonders bevorzugte Gruppen X leiten sich von Derivaten des Indolins, wie dem 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 5-Hydroxyindolin, 6-Hydroxyindolin, 5-Aminoindolin, 6-Aminoindolin und 4-Aminoindolin ab.

Ganz besonders bevorzugt sind Derivate des 5,6-Dihydroxyindolins der Formel (IIa), 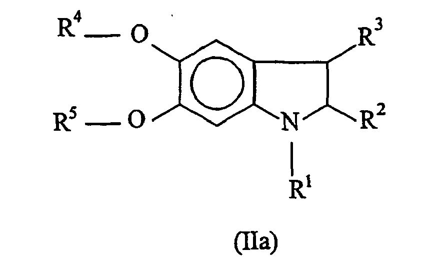 in der unabhängig voneinander
- R¹: steht für Wasserstoff, eine C1-C4-Alkylgruppe, eine C1-C4-Hydroxyakylgruppe, eine C3-C6-Cycloalkylgruppe, eine Vinylgruppe oder eine Allylgruppe,
- R²: steht für Wasserstoff oder eine -COOH-Gruppe, wobei die -COOH-Gruppe auch als Salz mit einem physiologisch verträglichen Kation vorliegen kann,
- R³: steht für Wasserstoff oder eine C1-C4-Alkylgruppe,
- R⁴: R⁴ steht für Wasserstoff, eine C1-C4-Alkylgruppe oder eine Gruppe -CO-R⁶, in der R⁶ steht für eine C1-C4-Alkylgruppe oder eine gegebenenfalls substituierte Phenylgruppe, und
- R⁵: steht für eine der unter R⁴ genannten Gruppen.

Erfindungsgemäß bevorzugte Vertreter sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin. Der Grundkörper, das 5,6-Dihydroxyindolin, ist ganz besonders bevorzugt.

Erfindungsgemäß bevorzugte Indole, von denen sich die Gruppe X ableitet, sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 5-Hydroxyindol, 6-Hydroxyindol, 5-Aminoindol, 6-Aminoindol und 4-Aminoindol.

Besonders bevorzugt sind Derivate des 5,6-Dihydroxyindols der Formel (IIb), 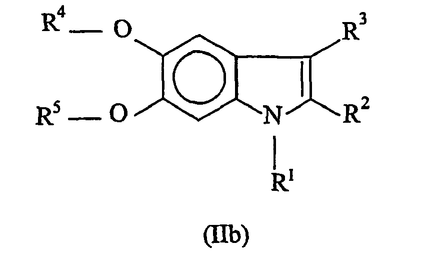 in der unabhängig voneinander
- R¹: steht für Wasserstoff, eine C1-C4-Alkylgruppe, eine C1-C4-Hydroxyalkylgruppe, eine C3-C6-Cycloalkylgruppe, eine Vinylgruppe oder Allylgruppe,
- R²: steht für Wasserstoff oder eine -COOH-Gruppe, wobei die -COOH-Gruppe auch als Salz mit einem physiologisch verträglichen Kation vorliegen kann,
- R³: steht für Wasserstoff, eine C1-C4-Alkylgruppe oder eine Gruppe -CH₂-NR⁷R⁸, in der R⁷ und R⁸ unabhängig voneinander stehen für Wasserstoff oder eine C1-C4-Alkylgruppe,
- R⁴: steht für Wasserstoff, eine C1-C4-Alkylgruppe oder eine Gruppe -CO-R⁶, in der R⁶ steht für eine C1-C4-Alkylgruppe oder eine gegebenenfalls substituierte Phenylgruppe, und
- R⁵: steht für eine der unter R⁴ genannten Gruppen.

Erfindungsgemäß bevorzugte Vertreter sind das 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol. Der Grundkörper, das 5,6-Dihydroxyindol, ist ganz besonders bevorzugt.

Die Gruppe Y steht für eine Gruppe, die abgeleitet ist von
- einem Oxidationsfarbstoffvorprodukt vom Kuppler- oder Entwicklertyp.

Gemäß einer ersten, bevorzugten Ausführungsform der vorliegenden Erfindung leitet sich die Gruppe Y ab von einem Oxidationsfarbstoffvorprodukt vom Kupplertyp.

Als Kupplerkomponenten werden in der Regel m-Phenylendiaminderivate, Naphthole, Resorcin und Resorcinderivate, Pyrazolone und m-Aminophenolderivate verwendet.

Bevorzugte Klassen von Oxidationsfarbstoffvorprodukten vom Kupplertyp, von denen sich die Gruppe Y ableiten kann, sind:
- 3-Aminophenol und dessen Derivate;
   Bevorzugte Vertreter sind 5-Amino-2-methylphenol, 5-(3-Hydroxypropylamino)-2-methylphenol, 3-Amino-2-chlor-6-methylphenol, 2-Hydroxy-4-aminophenoxy-ethanol, 3-Amino-6-methoxy-2-methylaminophenol, 2,6-Dimethyl-3-aminophenol, 3-Tri-fluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-Dimethylamino-phenol, 3-Diethylamino-phenol, N-Cyclopentyl-3-aminophenol, 1,3-Dihydroxy-5-(methylamino)-benzol, 3-Ethylamino-4-methylphenol und 2,4-Dichlor-3-aminophenol;
- 2-Aminophenol und dessen Derivate;
- 1,3-Diaminobenzol und dessen Derivate;
   Bevorzugte Vertreter sind 2,4-Diaminophenoxyethanol, 1,3-Bis-(2,4-diaminophenoxy)-propan, 1-Methoxy-2-amino-4-(2-hydroxyethylamino)benzol, 1,3-Bis-(2,4-diaminophenyl)-propan, 2,6-Bis-(2-hydroxyethylamino)-1-methylbenzol, 1-Amino-3-bis-(2-hydroxyethyl)-aminobenzol, 1,2-Bis-(2,4-diaminophenoxy)-benzol und 1,3-Bis-(2,4-diaminophenoxy)-benzol;
- 1,2-Diaminobenzol und dessen Derivate;
   Bevorzugte Vertreter sind 3,4-Diaminobenzoesäure und 2,3-Diamino-1-methyl-benzol;
- Di- und Trihydroxybenzole und deren Derivate;
   Bevorzugte Vertreter sind Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol sowie weiterhin Resorcindimethylether;
- Pyridinderivate;
   Bevorzugte Vertreter sind 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-3,4-diaminopyridin, 2,6-Dihydroxy-4-methylpyridin, 2,6-Diaminopyridin, 2,3-Diamino-6-methoxypyridin und 3,5-Diamino-2,6-dimethoxypyridin;
- Naphthalinderivate;
   Bevorzugte Vertreter sind 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin sowie weiterhin 1-Aminonaphthalin;
- Morpholinderivate;
   Bevorzugte Vertreter sind 6-Hydroxybenzomorpholin und 6-Aminobenzomorpholin;
- Chinoxalinderivate;
   Ein bevorzugter Vertreter ist 6-Methyl-1,2,3,4-tetrahydrochinoxalin;
- Pyrazolderivate;
   Ein bevorzugter Vertreter ist 1-Phenyl-3-methylpyrazol-5-on;
- Indolderivate;
   Bevorzugte Vertreter sind 4-Hydroxyindol, 6-Hydroxyindol und 7-Hydroxyindol;
- Methylendioxybenzolderivate;
   Bevorzugte Vertreter sind 3,4-Methylendioxyphenol, 1-Amino-3,4-methylendioxy-benzol und 1-(2'-Hydroxyethyl)-amino-3,4-methylendioxybenzol;
- Pyrimidinderivate;
   Bevorzugte Vertreter sind 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin;
- Heterocyclische Verbindungen, wie sie in den Offenlegungsschriften WO 97/35550, WO 97/35552, WO 97/35553, WO 98/08485 und WO 98/08486, auf die ausdrücklich Bezug genommen wird, offenbart sind.

Besonders bevorzugte Kupplersubstanzen sind hierbei 3-Aminophenol, 5-Amino-2-methylphenol, 2,4-Dichlor-3-aminophenol, 2-Chlor-6-methyl-3-aminophenol, 2-Methyl-4-chlor-5-aminophenol, 1,3-Phenylendiamin, 1,3-Bis-(2',4'-diaminophenoxy)-propan, Resorcin, Resorcinmonomethylether, 2-Methylresorcin, 5-Methylresorcin, 2,5-Dimethylresorcin, 2-Chlor-resorcin, 4-Chlor-resorcin, 1-Naphthol, 1,5-, 2,7- und 1,7-Dihydroxynaphthalin, 1-Phenyl-3-methyl-pyrazol-5-on, 2-Amino-3-hydroxypyridin und 2,6-Dihydroxy-3,4-dimethylpyridin.

Bezüglich weiterer Oxidationsfarbstoffvorprodukte vom Kupplertyp, von denen sich die Gruppe Y ableiten kann, wird weiterhin ausdrücklich auf die bekannten Monographien, z. B. Ch. Zviak, The Science of Hair Care, Kapitel 8 (Seiten 264-267), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Gemäß einer zweiten, ebenfalls bevorzugten, Ausführurigsform der vorliegenden Erfindung leitet sich die Gruppe Y ab von einem Oxidationsfarbstoffvorprodukt vom Entwicklertyp.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt.

Bevorzugte Klassen von Oxidationsfarbstoffvorprodukten vom Entwicklertyp, von denen sich die Gruppe Y ableiten kann, sind:
- 1,4-Diaminobenzol und dessen Derivate;
   Bevorzugte Vertreter sind p-Phenylendiamin, p-Toluylendiamin, 1-(2'-Hydroxyethyl)-2,5-diaminobenzol, N,N-Bis-(2-hydroxy-ethyl)-p-phenylendiamin, Bis-(4-aminophenyl)amin, 2-(2,5-Diaminophenoxy)-ethanol, 1,3-Bis(N(2-hydroxyethyl)-N(4-aminophenylamino))-2-propanol, 1, 10-Bis-(2,5-diaminophenyl)-1,4,7,10-tetraoxadecan und N,N'-Bis-(4'-aminophenyl)-1,4-diazacycloheptan sowie entsprechende Verbindungen mit einem oder mehreren Halogenatomen, insbesondere Chlor und Fluor, am Benzolring;
- 1,2-Diaminobenzol und dessen Derivate;
- 1,2,4-Triaminobenzol und dessen Derivate;
- 4-Aminophenol und dessen Derivate;
   Bevorzugte Vertreter sind p-Aminophenol, 2-Chlor-4-aminophenol, 4-Amino-3-methylphenol, 2-Hydroxyethylamino-4-amino-phenol, 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, 4-Amino-2-(2-hydroxyethoxy)-phenol;
- 2-Aminophenol und dessen Derivate;
   Bevorzugte Vertreter sind o-Aminophenol und 2-Amino-5-methylphenol;
- Diaminopyridinderivate;
   Bevorzugte Vertreter sind 2,5-Diaminopyridin, 2,5-Diamino-4-methylpyridin, 2,5-Diamino-4,6-dimethylpyridin;
- Triaminopyridinderivate:
   ein bevorzugter Vertreter ist 2,3,5-Triaminopyridin;
- heterocyclische Hydrazone;
- 4-Aminopyrazolderivate;
   Bevorzugte Vertreter sind 4,5-Diamino-1,3-dimethylpyrazol, 1-Phenyl-3-carboxyamido-4-amino-pyrazolon-5 sowie 4,5-Diaminopyrazol-Derivate nach EP 0 740 741 bzw. WO 94/08970 wie z. B. 4,5-Diamino-1-(2'-hydroxyethyl)-pyrazol und 4,5-Diamino-1-(4'-chlorbenzyl)-pyrazol;
- Pyrimidin-Derivate;
   Bevorzugte Vertreter sind 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diamino-pyrimidin und 2-Dimethylamino-4,5,6-triaminopyrimidin.

Besonders bevorzugte Entwicklersubstanzen sind hierbei p-Phenylendiamin, p-Toluylendiamin, 1,2,4-Phenylentriamin, p-Aminophenol, 1-(2'-Hydroxyethyl)-2,5-diaminobenzol, N,N-Bis-(2'-hydroxyethyl)-p-phenylendiamin, 2-(2',5'-Diaminophenyl)-ethanol, N,N'-Bis-(2'-hydroxyethyl)-N,N'-bis-(4'-aminophenyl)-1,3-diamino-propan-2-ol, 2-(2',5'-Diaminophenoxy)-ethanol, 4-Amino-3-methylphenol, 4-Amino-2-((diethylamino)-methyl)-phenol, 2-Aminomethyl-4-aminophenol, 2,5-Diaminopyridin, 2,5-Diamino-4-methylpyridin, 2,5-Diamino-4,6-dimethylpyridin, 2,3,5-Triaminopyridin 2,4,5,6-Tetraaminopyrimidin, 2-Hydroxy-4,5,6-triaminopyrimidin, 2,4-Dihydroxy-5,6-diaminopyrimidin, 4-Hydroxy-2,5,6-triaminopyrimidin und 4,5-Diamino-1,3-dimethylpyrazol, 4,5-Diamino-1-(2'-hydroxyethyl)-pyrazol.

Ferner kann es erfindungsgemäß bevorzugt sein, daß sich die Gruppe Y von einer Entwicklerkomponente ableitet, die ein p-Phenylendiaminderivat oder eines seiner physiologisch verträglichen Salze darstellt. Besonders bevorzugt sind p-Phenylendiaminderivate der Formel (E1) 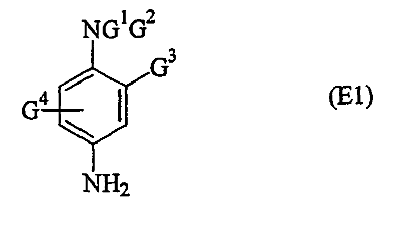 wobei
- G¹ steht für ein Wasserstoffatom, einen C₁- bis C₄-Alkylrest, einen C₁- bis C₄₋Monohydroxyalkylrest, einen C₂- bis C₄-Polyhydroxyalkylrest, einen (C₁- bis C₄)-Alkoxy-(C₁- bis C₄)-alkylrest, einen 4'-Aminophenykest oder einen C₁- bis C₄₋Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄₋Monohydroxyalkylrest, einen C₂- bis C₄-Polyhydroxyalkylrest, einen (C₁- bis C₄)-Alkoxy-(C₁- bis C₄)-alkykest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, Iod- oder Fluoratom, einen C₁- bis C₄-Alkykest, einen C₁- bis C₄-Monohydroxyalkylrest, einen C₂-bis C₄-Polyhydroxyalkylrest, einen C₁- bis C₄-hydroxyalkoxyrest, einen C₁₋bis C₄-Acetylaminoalkoxyrest, einen C₁- bis C₄- Mesylaminoalkoxyrest oder einen C₁- bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Methylen- oder Ethylendioxygruppe bilden.

Beispiele für die als Substituenten in den erfindungsgemäßen Verbindungen genannten, C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄₋Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Ein Beispiel für eine C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (II) sind insbesondere die Aminogruppen, C₁- bis C₄-Monoalkylaminogruppen, C₁- bis C₄-Dialkylaminogruppen, C₁- bis C₄₋Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenylp-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin und N,N-Bis-(β-hydroxyethyl)-p-phenylendiamin.

Es kann erfindungsgemäß weiterhin bevorzugt sein, daß sich die Gruppe Y von einer Entwicklerkomponente ableitet, die eine Verbindung darstellt, die wenigstens zwei aromatische Kerne enthält, die mit Amino- und/oder Hydroxylgruppen substituiert ist.

Unter diesen zweikernigen Entwicklerkomponenten, kann man insbesondere die Verbindungen nennen, die der folgenden Formel (E2) entsprechen, sowie ihre physiologisch verträglichen Salze: 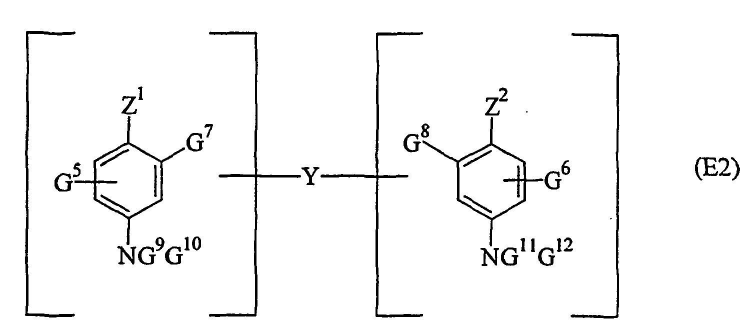 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄₋Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄₋Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄₋Alkylrest,
mit den Maßgaben, daß
- die Verbindungen der Formel (E2) nur eine Verbrückung Y pro Molekül enthalten und
- die Verbindungen der Formel (E2) wenigstens eine Aminogruppe enthalten, die wenigstens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4'-aminophenyl)-1,3-diamino-propan-2-ol, Bis-(2-hydroxy-5-aminophenyl)-methan, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, daß sich die Gruppe Y von einer Entwicklerkomponente ableitet, die ein p-Aminophenolderivat oder eines seiner physiologisch verträglichen Salze darstellt. Besonders bevorzugt sind p-Aminophenolderivate der Formel (E3) 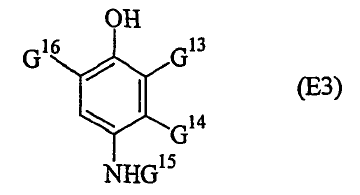 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁- bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest oder einen C₁- bis C₄-Cyanoalkylrest,
- G¹⁵ steht für Wasserstoff, einen C₁- bis C₄-Alkylrest, einen C₁- bis C₄₋Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol und 4-Amino-2-(diethylaminomethyl)-phenol.

Ferner kann die Entwicklerkomponente ausgewählt sein aus o-Aminophenol und seinen Derivaten, wie beispielsweise 2-Amino-4-methylphenol, 2-Amino-4-chlorphenol oder 2-Amino-5-methylphenol.

Weiterhin kann sich die Gruppe Y von einer heterocyclischen Entwicklerkomponente ableiten, wie beispielsweise Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4'-Methoxyphenyl)-amino-3-amino-pyridin, 2,3-Diamino-6-methoxy-pyridin, 2-(β-Methoxyethyl)-amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydröxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)-amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamko-4-(β-hydroxyethyl)-amino-1-methylpyrazol.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht: 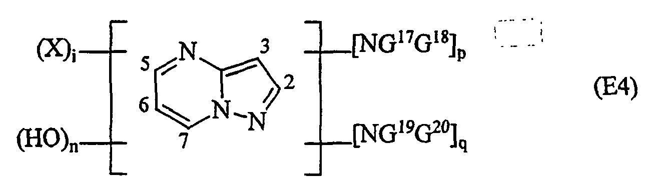 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen SulfonylRest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄₋Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄₋hydroxyalkyl)-aminoalkylrest, einen Aminorest, einen C₁- bis C₄-Alkyl- oder Di-(C₁- bis C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, daß
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

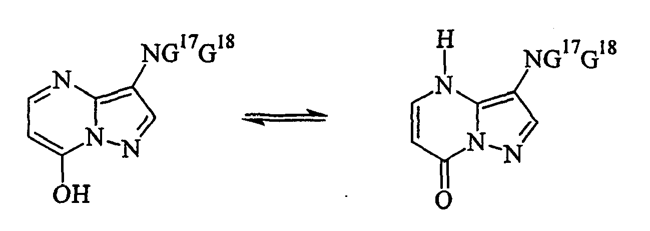

Unter den Pyrazol-[1,5-a]-pyrimidinen der obenstehenden Formel (E4) kann man insbesondere nennen:
- Pyrazol-[1,5-a]-pyrimidin-3,7-diamin;
- 2,5-Dimethyl-pyrazol-[1,5-a]-pyrimidin-3,7-diamin;
- Pyrazol-[1,5-a]-pyrimidin-3,5-diamin;
- 2,7-Dimethyl-pyrazol-[1,5-a]-pyrimidin-3,5-diamin;
- 3-Aminopyrazol-[1,5-a]-pyrimidin-7-ol;
- 3-Aminopyrazol-[1,5-a]-pyrimidin-5-ol;
- 2-(3-Aminopyrazol-[1,5-a]-pyrimidin-7-ylamino)-ethanol;
- 2-(7-Aminopyrazol-[1,5-a]-pyrimidin-3-ylamino)-ethanol;
- 2-[(3-Aminopyrazol-[1,5-a]-pyrimidin-7-yl)-(2-hydroxy-ethyl)-amino]-ethanol;
- 2-[(7-Aminopyrazol-[1,5-a]-pyrimidin-3-yl)-(2-hydroxy-ethyl)-amino]-ethanol;
- 5,6-Dimethylpyrazol-[1,5-a]-pyrimidin-3,7-diamin;
- 2,6-Dimethylpyrazol-[1,5-a]-pyrimidin-3,7-diamin;
- 3-Amino-7-dimethylamino-2,5-dimethylpyrazol-[1,5-a]-pyrimidin;
sowie ihre physiologisch verträglichen Salze und ihre tautomeren Formen, wenn ein tautomeres Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

Bezüglich weiterer Oxidationsfarbstoffvorprodukte vom Entwicklertyp, von denen sich die Gruppe Y ableiten kann, wird weiterhin ausdrücklich auf die bekannten Monographien,
z. B. Ch. Zviak, The Science of Hair Care, Kapitel 8 (Seiten 264-267), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Die Spacergruppe S der Formel (I) kann ein Strukturelement sein, das ein gemeinsamer Bestandteil der Gruppen X und Y ist. Im einfachsten Fall handelt es sich bei dem Strukturelement um ein Atom, welches gemeinsamer Bestandteil der Gruppe X und Y ist, vorzugsweise handelt es sich bei dem Atom um ein C-, O-, S-, N- oder P-Atom. Besonders bevorzugt handelt es sich um ein N enthaltendes Strukturelement, und noch bevorzugter um ein N enthaltendes Strukturelement gemäß Formel (III), welches ein gemeinsamer Bestandteil der Gruppe X abgeleitet von Indol- oder Indolinderivaten und der Gruppe Y abgeleitet von Aminobenzol- oder Pyrimidin- oder -Pyridinderivaten ist.

Bevorzugt steht die Gruppe S in der Strukturformel (I) aber für wenigstens eine Spacergruppe. Wenn mehrere Spacergruppen vorliegen, beispielsweise zwei Spacergruppen, sind diese vorzugsweise unabhängig voneinander eine unsubstituierte und/oder substituierte Alkylengruppe, die jeweils endständig an Stickstoffatom(e) der Gruppen X und Y angreifen.

Gemäß einer dritten Ausführungsform der Erfindung steht die Gruppe S in der Strukturformel (I) für eine direkte Bindung.

In diesen Fällen, in denen die Gruppe S in der Strukturformel (I) für eine direkte Bindung steht, wird in den meisten Fällen eine Wechselwirkung der π-Elektronen-Systeme der Gruppen X und Y auftreten, so daß in der Regel das Lichtabsorptionsverhalten des Hybridfarbstoffes sich von dem der Gruppen X und Y deutlich unterscheidet. Dadurch wird auf dem Haar ein signifikant veränderter Farbton erzielt im Vergleich zu Ausfärbungen, die mit einer Mischung entsprechender Farbstoffe durchgeführt werden, die den Gruppen X und Y entsprechen.

Ein Kerngedanke der vorliegenden Erfindung ist jedoch, die bei der Verwendung komplexer Farbstoffmischungen in vielen Bereichen, z.B. hinsichtlich Aufziehvermögen und Waschechtheit, auftretenden Probleme zu beheben, ohne den Farbton und die Nuance zu verändern.
Es ist daher in der Regel erfindungsgemäß bevorzugt, daß S für eine Spacer-Gruppe(n) steht, über die keine Wechselwirkung der π-Elektronensysteme der Gruppen X und Y stattfindet. Bevorzugt enthält daher S wenigstens ein Kohlenstoffatom mit sp³⁻Hybridisierung auf der direkten Verbindungslinie zwischen den Gruppen X und Y.

Wenn mehrere Spacergruppen vorliegen, beispielsweise zwei Spacergruppen, sind diese vorzugsweise unabhängig voneinander eine unsubstituierte oder substituierte Alkylgruppe, die jeweils endständig an Stickstoffatome der Gruppen X und Y angreifen.

Bevorzugte Spacergruppen S sind:
- Alkylengruppen der allgemeinen Formel -CₙH₂ₙ-, insbesondere -(CH₂)ₙ-, in denen n für eine ganze Zahl, insbesondere eine Zahl von 1 bis 8 und ganz besonders bevorzugt für eine Zahl von 1 bis 4, steht;
   Erfindungsgemäß bevorzugte Alkylengruppen sind die Methylen-, 1,2-Ethylen- und 1,3-Propylen-Gruppe;
- Cycloaliphatische Gruppen wie Cyclopentyl-, Cyclohexyl- und Cycloheptylgruppen;
- Mono- und Polyhydroxyalkylengruppen der allgemeinen Formel -CₙH₂ₙ₋ₓ(OH)ₓ, in denen n für eine ganze Zahl, insbesondere eine Zahl von 1 bis 8 und ganz besonders bevorzugt für eine Zahl von 1 bis 4, steht und x für eine ganze Zahl, insbesondere für eine Zahl von 1 bis 3, steht;
   Bevorzugte Hydroxyalkylengruppen sind die Hydroxymethylen-, die Hydroxy-1,2-ethylen-, die 2-Hydroxy-1,3-propylen, die 2,3-Dihydroxy-1,3-propylen- und die 2,3-Dihydroxy-1,4-butylen-Gruppe;
- gegebenenfalls an den Alkylketten substituierte Dialkylenaminogruppen, insbesondere solche der allgemeinen Formel -(CH₂)ₙ-N(Z)-(CH₂)ₘ-,
   in der n und m unabhängig voneinander für eine ganze Zahl von 1 bis 8, insbesondere von 1 bis 4, stehen können, jedoch bevorzugt für die gleiche Zahl stehen, und Z für Wasserstoff, eine C₁₋₈-, insbesondere C₁₋₄-Alkylgruppe, eine C₁₋₈-, insbesondere C₁₋₄₋Monohydroxyalkylgruppe, eine C₂₋₈-, insbesondere C₂₋₄-Dihydroxyalkylgruppe oder eine eine C₃₋₈-, insbesondere C₃₋₄-Trihydroxyalkylgruppe steht, sowie solche der allgemeinen Formel 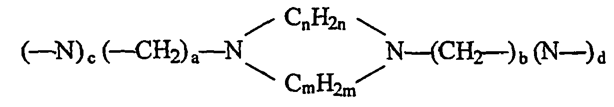 in der a und b unabhängig voneinander für ganze Zahlen von 0 bis 4 und c und d für 0 oder 1 stehen mit der Maßgabe, daß c = 0 ist, wenn a = 0 ist und d = 0 ist, wenn b = 0 ist, n für eine ganze Zahl von 1 bis 5 und m für eine ganze Zahl von 1 bis 3 steht mit der Maßgabe, daß die Summe n+m 3 bis 8 ist. Besonders bevorzugt ist die 1,4-Piperazinogruppe;
   gegebenenfalls an den Alkylketten substituierte Trialkylendiaminogruppen, insbesondere solche der allgemeinen Formel -(CH₂)ₙ-N(Z)-(CH₂)ₘ-N(A)-(CH₂)ₚ-, in der n, m und p unabhängig voneinander für eine ganze Zahl von 1 bis 8, insbesondere von 1 bis 4, stehen können, n und m jedoch bevorzugt für die gleiche Zahl stehen, und Z und A unabhängig voneinander für Wasserstoff, eine C₁₋₈-, insbesondere C₁₋₄₋Alkylgruppe, eine C₁₋₈-, insbesondere C₁₋₄-Monohydroxyalkylgruppe, eine C₂₋₈-, insbesondere
   C₂₋₄-Dihydroxyalkylgruppe oder eine eine C₃₋₈-, insbesondere C₃₋₄-Trihydroxyalkylgruppe stehen;
- gegebenenfalls an den Alkylketten substituierte Ethergruppen, insbesondere solche der allgemeinen Formel -(CH₂)ₙ-O-(CH₂)ₘ-, in der n und m unabhängig voneinander für eine ganze Zahl von 1 bis 8, insbesondere von 1 bis 4, stehen können, jedoch bevorzugt für die gleiche Zahl stehen;
- gegebenenfalls an den Alkylketten substituierte Polyethergruppen, insbesondere solche der allgemeinen Formel -(CH₂)ₙ-O-(CH₂)ₘ-O-(CH₂)ₘ-O-(CH₂)ₙ-, in der n und m unabhängig voneinander für eine ganze Zahl von 1 bis 8, insbesondere von 1 bis 4, stehen können, jedoch bevorzugt für die gleiche Zahl stehen;
- schwefelhaltige Gruppen, insbesondere Gruppen der allgemeinen Formel -(CH₂)ₙS(O)ₒ-(CH₂)ₘ-, in der n und m unabhängig voneinander für eine ganze Zahl von 1 bis 8, insbesondere von 1 bis 4, stehen können, jedoch bevorzugt für die gleiche
   Zahl stehen, und o für 0, 1 oder 2 steht.

Die Spacer S sind in den erfindungsgemäßen Hybrid-Farbstoffen über ihre beiden freien Bindungen mit den Gruppen X und Y so verknüpft, daß sie jeweils als Substituent an die Stelle eines Wasserstoffatoms der Farbstoff- bzw. Farbstoffvorprodukt-Moleküle treten, die den Gruppen X und Y zugrunde liegen.

Gemäß einer ersten bevorzugten Ausführungsform tritt die Spacergruppe S als Substituent an die Stelle eines Wasserstoffatoms, das direkt an ein Ringsystem der Gruppe X oder Y gebunden ist. Beispiele für solche Ringsysteme sind
- aromatische und cycloaliphatische Kohlenwasserstoff-Ringsysteme, insbesondere Benzol-, Naphthalin-, Anthracen-, Naphthochinon- und Anthrachinon-Systeme;
- heterocyclische Ringsysteme, insbesondere Pyridin-, Pyrazol-, Pyrimidin-, Indol- und Indolinsysteme.

Gemäß einer zweiten, bevorzugten Ausführungsform tritt die Spacergruppe S als Substituent an die Stelle eines Wasserstoffatoms einer primären oder sekundären Aminogruppe, die direkt oder über eine aliphatische Kohlenwasserstoffgruppe an ein aromatisches, cycloaliphatisches oder heterocyclisches Ringsystem gebunden ist.

Gemäß einer dritten Ausführungsform tritt die Spacergruppe S als Substituent an die Stelle des Wasserstoffatoms einer Hydroxygruppe, die direkt oder über eine aliphatische Kohlenwasserstoffgruppe an ein aromatisches, cycloaliphatisches oder heterocyclisches Ringsystem gebunden ist.

Besonders bevorzugt im Rahmen der vorliegenden Erfindung sind Hybridfarbstoffe der allgemeinen Formel (III), bei denen sich X von Indol- oder Indolinderivaten als Vorläufer des Melanins und Y von Aminobenzol- oder -pyridinderivaten ableiten und S ein N-Atom enthaltendes Strukturelement ist, das ein gemeinsamer Bestandteil der Gruppen X und Y ist: 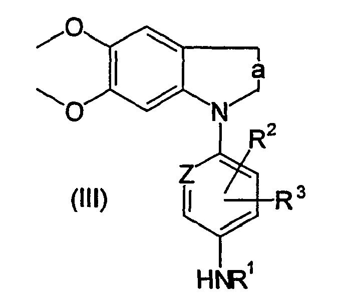 wobei R¹ H, C₁₋₄-Alkyl, C₂₋₄-Alkenyl, C₁₋₄-Hydroxyalkyl oder C₂₋₄-Polyhydroxyalkyl und R² und R³ gleich oder unabhängig voneinander H, C₁₋₄-Alkyl, Halogen, C₁₋₄-Alkoxy, Amino-C₁₋₄-alkyl oder N(R¹)₂ sind, Z für CH oder N steht und a eine Einfach- oder Doppelbindung darstellt. So sind z. B. folgende Verbindungen besonders bevorzugt:

| | R¹ | R² | R³ | Z | a |
|---|---|---|---|---|---|
| III a | H | H | H | CH | Einfach |
| III b | H | H | H | N | Einfach |

Die erfindungsgemäßen Hybridfarbstoffe eignen sich hervorragend zum Färben keratinischer Fasern. Unter keratinischen Fasern sind dabei Pelze, Wolle, Federn und insbesondere menschliche Haare zu verstehen. Einer Verwendung auch auf anderen Gebieten, insbesondere in der Farbphotographie, steht aber nichts entgegen.

Ein zweiter Gegenstand der vorliegenden Erfindung sind daher Mittel zum Färben keratinischer Fasern, insbesondere menschlicher Haare, die einen Hybridfarbstoff gemäß Struktur (I) enthalten. Selbstverständlich umfaßt die erfindungsgemäße Lehre auch solche Mittel, die Kombinationen von mehr als einem Hybridfarbstoff der Struktur (I) enthalten.

Die erfindungsgemäßen Mittel zum Färben menschlicher Haare können weiterhin alle für solche Mittel üblichen Bestandteile enthalten.

Die erfindungsgemäßen Mittel enthalten die Hybridfarbstoffe der Struktur (I) üblicherweise in Mengen von 0,01 Gew.-% - 10 Gew.-%, bezogen auf das gesamte Färbemittel. Mengen von 0,05 Gew.-% - 5 Gew.-%, insbesondere Mengen von 0,1 Gew.-% - 3 Gew.-%, sind bevorzugt.

Bevorzugt enthalten die erfindungsgemäßen Färbemittel wenigstens einen weiteren Farbstoff, ein weiteres Farbstoffvorprodukt und/oder ein Indol- oder Indolin-Derivat als Melanin-Vorstufe.

In einer ersten Ausführungsform sind solche Färbemittel besonders bevorzugt, die neben einem Hybridfarbstoff der Struktur (I) wenigstens ein Oxidationsfarbstoffvorprodukt enthalten. Diese Oxidationsfarbstoffvorprodukte können sowohl vom Kupplertyp als auch vom Entwicklertyp sein.

Als Oxidationsfarbstoffvorprodukte vom Entwicklertyp kommen beispielsweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate in Betracht.

Erfindungsgemäß bevorzugte Klassen von Entwicklerkomponenten sind:
- 1,4-Diaminobenzol und dessen Derivate;
   Bevorzugte Vertreter sind p-Phenylendiamin, p-Toluylendiamin, 1-(2'-Hydroxyethyl)-2,5-diaminobenzol, N,N-Bis-(2-hydroxyethyl)-p-phenylendiamin, Bis-(4-aminophenyl)amin, 2-(2,5-Diaminophenoxy)-ethanol, 1,3-Bis(N(2-hydroxyethyl)-N(4-aminophenylamino))-2-propanol, 1,10-Bis-(2,5-diaminophenyl)-1,4,7,10-tetraoxadecan und N,N'-Bis-(4'-aminophenyl)-1,4-diazacycloheptan sowie entsprechende Verbindungen mit einem oder mehreren Halogenatomen, insbesondere Chlor und Fluor, am Benzolring;
- 1,2-Diaminobenzol und dessen Derivate;
- 1,2,4-Triaminobenzol und dessen Derivate;
- 4-Aminophenol und dessen Derivate;
   Bevorzugte Vertreter sind p-Aminophenol, 2-Chlor-4-aminophenol, 4-Amino-3-methylphenol, 2-Hydroxyethylamino-4-amino-phenol, 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, 4-Amino-2-(2-hydroxyethoxy)-phenol;
- 2-Aminophenol und dessen Derivate;
   Ein bevorzugter Vertreter ist o-Aminophenol;
- Diaminopyridinderivate;
   Bevorzugte Vertreter sind 2,5-Diaminopyridin, 2,5-Diamino-4-methylpyridin, 2,5-Diamino-3-methylenamino-4,6-dimethylpyridin;
- Triaminopyridinderivate;
   ein bevorzugter Vertreter ist 2,3,5-Triaminopyridin;
- heterocyclische Hydrazone;
- 4-Aminopyrazolderivate;
   Bevorzugte Vertreter sind 4,5-Diamino-1,3-dimethylpyrazol, 1-Phenyl-3-carboxyamido-4-amino-pyrazolon-5 sowie 4,5-Diaminopyrazol-Derivate nach EP 0 740 741 bzw. WO 94/08970 wie z. B. 4,5-Diamino-1-(2'-hydroxyethyl)-pyrazol und 4,5-Diamino-1-(4'-chlorbenzyl)-pyrazol;
- Pyrimidin-Derivate;
   Bevorzugte Vertreter sind 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diamino-pyrimidin und 2-Dimethylamino-4,5,6-triaminopyrimidin.

Besonders bevorzugte Entwicklertypen bzw. Substanzen sind hierbei diese der vorgenannten Formeln E1, E2, E3 und E4, sowie insbesondere p-Phenylendiamin, p-Toluylendiamin, 1,2,4-Triaminobenzol, p-Aminophenol, 1-(2'-Hydroxyethyl)-2,5-diaminobenzol, N,N-Bis-(2'-hydroxyethyl)-p-phenylendiamin, 2-(2',5'-Diaminophenyl)-ethanol, N,N'-Bis-(2'-hydroxyethyl)-N,N'-bis-(4'-aminophenyl)-1,3-diamino-propan-2-ol, 2-(2',5'-Diaminophenoxy)-ethanol, 4-Amino-3-methylphenol, 4-Amino-2-((diethylamino)-methyl)-phenol, 2-Aminomethyl-4-aminophenol, 2,5-Diaminopyridin, 2,5-Diamino-4-methylpyridin, 2,5-Diamino-3-methylenamino-4,6-dimethylpyridin, 2,3,5-Triaminopyridin 2,4,5,6-Tetraaminopyrimidin, 2-Hydroxy-4,5,6-triaminopyrimidin, 2,4-Dihydroxy-5,6-diaminopyrimidin, 4-Hydroxy-2,5,6-triaminopyrimidin und 4,5-Diamino-1,3-dimethylpyrazol, 4,5-Diamino-1-(2'-hydroxyethyl)-pyrazol.

Als Oxidationsfarbstoffvorprodukte vom Kupplertyp kommen beispielsweise m-Phenylendiaminderivate, Naphthole, Resorcin und Resorcinderivate, Pyrazolone und m-Aminophenolderivate in Betracht.

Erfindungsgemäß bevorzugte Klassen von Kupplerkomponenten sind:
- 3-Aminophenol und dessen Derivate;
   Bevorzugte Vertreter sind 5-Amino-2-methylphenol, 5-(3-Hydroxypropylamino)-2-methylphenol, 3-Amino-2-chlor-6-methylphenol, 2-Hydroxy-4-aminophenoxyethanol, 3-Amino-6-methoxy-2-methylaminophenol, 2,6-Dimethyl-3-aminophenol, 3-Tri-fluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-Dimethylamino-phenol, 3-Diethylamino-phenol, N-Cyclopentyl-3-aminophenol, 1,3-Dihydroxy-5-(methylamino)-benzol, 3-Ethylamino-4-methylphenol und 2,4-Dichlor-3-aminophenol;
- 2-Aminophenol und dessen Derivate;
- 1,3-Diaminobenzol und dessen Derivate; Bevorzugte Vertreter sind 2,4-Diaminophenoxyethanol, 1,3-Bis-(2,4-diaminophenoxy)-propan, 1-Methoxy-2-amino-4-(2-hydroxyethylamino)benzol, 1,3-Bis-(2,4-diaminophenyl)-propan, 2,6-Bis-(2-hydroxyethylamino)-1-methylbenzol, 1-Amino-3-bis-(2-hydroxyethyl)-aminobenzol, 1,2-Bis-(2,4-diaminophenoxy)-benzol und 1,3-Bis-(2,4-diaminophenoxy)-benzol;
- 1,2-Diaminobenzol und dessen Derivate;
   Bevorzugte Vertreter sind 3,4-Diaminobenzoesäure und 2,3-Diamino-1-methyl-benzol;
- Di- und Trihydroxybenzole und deren Derivate;
   Bevorzugte Vertreter sind Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol sowie weiterhin Resorcindimethylether;
- Pyridinderivate;
   Bevorzugte Vertreter sind 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-3,4-diaminopyridin, 2,6-Dihydroxy-4-methylpyridin, 2,6-Diaminopyridin, 2,3-Diamino-6-methoxypyridin und 3,5-Diamino-2,6-dimethoxypyridin;
- Naphthalinderivate;
   Bevorzugte Vertreter sind 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin sowie weiterhin 1-Aminonaphthalin;
- Morpholinderivate;
   Bevorzugte Vertreter sind 6-Hydroxybenzomorpholin und 6-Aminobenzomorpholin;
- Chinoxalinderivate;
   Ein bevorzugter Vertreter ist 6-Methyl-1,2,3,4-tetrahydrochinoxalin;
- Pyrazolderivate;
   Ein bevorzugter Vertreter ist 1-Phenyl-3-methylpyrazol-5-on;
- Indolderivate;
   Bevorzugte Vertreter sind 4-Hydroxyindol, 6-Hydroxyindol und 7-Hydroxyindol;
- Methylendioxybenzolderivate;
   Bevorzugte Vertreter sind 3,4-Methylendioxyphenol, 1-Amino-3,4-methylendioxy-benzol und 1-(2'-Hydroxyethyl)-amino-3,4-methylendioxybenzol;
- Pyrimidinderivate;
   Bevorzugte Vertreter sind 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin;
- Heterocyclische Verbindungen, wie sie in den Offenlegungsschriften WO 97/35550,
   WO 97/35552, WO 97/35553, WO 98/08485 und WO 98/08486, auf die ausdrücklich Bezug genommen wird, offenbart sind.

Besonders bevorzugte Kupplersubstanzen sind hierbei 3-Aminophenol, 5-Amino-2-methylphenol, 2,4-Dichlor-3-aminophenol, 2-Chlor-6-methyl-3-aminophenol, 2-Methyl-4-chlor-5-aminophenol, 1,3-Phenylendiamin, 1,3-Bis-(2',4'-diaminophenoxy)-propan, Resorcin, Resorcinmonomethylether, 2-Methylresorcin, 5-Methylresorcin, 2,5-Dimethylresorcin, 2-Chlor-resorcin, 4-Chlor-resorcin, 1-Naphthol, 1,5-, 2,7- und 1,7-Dihydroxynaphthalin, 1-Phenyl-3-methyl-pyrazol-5-on, 2-Amino-3-hydroxypyridin und 2,6-Dihydroxy-3,4-dimethylpyridin.

Im Rahmen dieser Ausführungsform kann bevorzugt sein:
- Hybridfarbstoff der Struktur (I) mit einer Gruppe Y, abgeleitet von einem Oxidationsfarbstoffvorprodukt vom Kupplertyp, in Kombination mit wenigstens einem weiteren Oxidationsfarbstoffvorprodukt vom Entwicklertyp
- Hybridfarbstoff der Struktur (I) mit einer Gruppe Y, abgeleitet von einem Oxidationsfarbstoffvorprodukt vom Entwicklertyp, in Kombination mit wenigstens einem weiteren Oxidationsfarbstoffvorprodukt vom Kupplertyp.

Die erfindungsgemäßen Mittel enthalten die weiteren Oxidationsfarbstoff-Vorprodukte vom Kuppler- und Entwicklertyp üblicherweise in Mengen von jeweils 0,01 Gew.-% - 10 Gew.-%, bezogen auf das gesamte Färbemittel. Mengen von 0,05 Gew.-% - 5 Gew.-%, insbesondere von 0,1 Gew.-% - 3 Gew.-%, sind bevorzugt.

In einer zweiten Ausführungsform sind solche Färbemittel bevorzugt, die neben einem Hybridfarbstoff der Struktur (I) wenigstens einen direktziehenden Farbstoff enthalten.

Solche erfindungsgemäß einzusetzenden direktziehenden Farbstoffe sind beispielsweise Nitrophenylendiamine, Nitroaminophenole, Azofarbstoffe, Anthrachinone und Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, HC Orange 1, Disperse Orange 3, HC Red 1, HC Red 3, HC Red 13, HC Red BN, Basic Red 76, HC Blue 2, HC Blue 12, Disperse Blue 3, Basic Blue 7, Basic Blue 26, Basic Blue 99, HC Violet 1, Disperse Violet 1, Disperse Violet 4, Basic Violet 2, Basic Violet 14, Acid Violet 43, Disperse Black 9, Acid Black 52, Basic Brown 16 und Basic Brown 17 bekannten Verbindungen sowie 1,4-Bis-(β-hydroxyethyl)-amino-2-nitrobenzol, 3-Nitro-4-(β-hydroxyethyl)-aminophenol, 4-Amino-2-nitrodiphenylamin-2'-carbonsäure, 6-Nitro-1,2,3,4-tetrahydrochinoxalin, 2-Hydroxy-1,4-naphthochinon, 1-(2'-Hydroxyethyl)amino-4-inethyl-2-nitrobenzol, Pikraminsäure, 2-Amino-6-chloro-4-nitrophenol, 4-Ethylamino-3-nitrobenzoesäure und 2-Chloro-6-ethylamino-1-hydroxy-4-nitrobenzol. Die kationischen direktziehenden Farbstoffe, die unter dem Warenzeichen Arianor^{®} vertrieben werden, sind besonders bevorzugte direktziehende Farbstoffe.

Weiterhin können die erfindungsgemäßen Mittel auch in der Natur vorkommende Farbstoffe enthalten, wie sie beispielsweise in Henna rot, Henna neutral, Henna schwarz, Kamillenblüte, Sandelholz, schwarzer Tee, Faulbaumrinde, Salbei, Blauholz, Krappwurzel, Catechu, Sedre und Alkannawurzel enthalten sind.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

In einer dritten Ausführungsform sind solche Färbemittel besonders bevorzugt, die neben einem Hybridfarbstoff der Struktur (I) wenigstens ein Derivat des Indols oder Indolins als Vorläufer des Melanins enthalten.

Erfindungsgemäß bevorzugt sind solche Indole und Indoline, die wenigstens eine Hydroxy- und/oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. Verbindungen mit zwei dieser Gruppen, insbesondere zwei Hydroxygruppen, von denen eine oder beide verethert oder verestert sind, sind besonders bevorzugt.

Erfindungsgemäß besonders bevorzugte Farbstoffvorprodukte sind Derivate des Indolins, wie das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 5-Hydroxyindolin, 6-Hydroxyindolin, 5-Aminoindolin, 6-Aminoindolin und 4-Aminoindolin.

Ganz besonders bevorzugt sind Derivate des 5,6-Dihydroxyindolins der Formel (IVa), 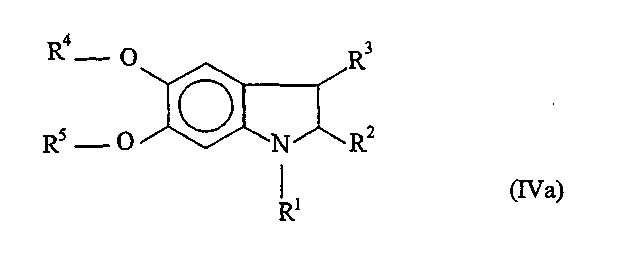 in der unabhängig voneinander
- R¹: steht für Wasserstoff, eine C1-C4-Alkylgruppe, eine C1-C4-Hydroxy-alkylgruppe oder eine C3-C6-Cycloalkylgruppe, eine Vinylgruppe oder eine Allylgruppe,
- R²: steht für Wasserstoff oder eine -COOH-Gruppe, wobei die -COOH-Gruppe auch als Salz mit einem physiologisch verträglichen Kation vorliegen kann,
- R³: steht für Wasserstoff oder eine C1-C4-Alkylgruppe,
- R⁴: steht für Wasserstoff, eine C1-C4-Alkylgruppe oder eine Gruppe -CO-R⁶, in der R⁶ steht für eine C1-C4-Alkylgruppe oder eine gegebenenfalls substituierte Phenylgruppe, und
- R⁵: steht für eine der unter R⁴ genannten Gruppen,
oder ein physiologisch verträgliches Salz dieser Verbindungen mit einer organischen oder anorganischen Säure.

Erfindungsgemäß bevorzugte Vertreter sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin. Der Grundkörper, das 5,6-Dihydroxyindolin, ist ganz besonders bevorzugt.

Erfindungsgemäß bevorzugte Indole sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 5-Hydroxyindol, 6-Hydroxyindol, 5-Aminoindol, 6-Aminoindol und 4-Aminoindol.

Besonders bevorzugt sind Derivate des 5,6-Dihydroxyindols der Formel (IVb), 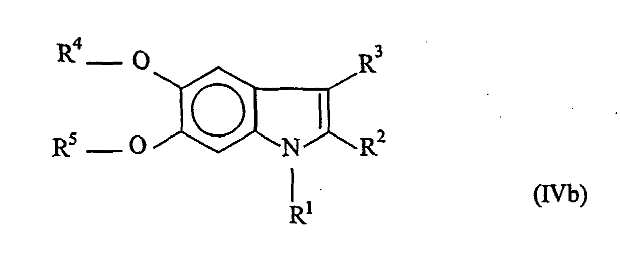 in der unabhängig voneinander
- R¹: steht für Wasserstoff, eine C1-C4-Alkylgruppe, eine C1-C4-Hydroxyalkylgruppe, eine C3-C6-Cycloalkylgruppe, eine Vinylgruppe oder eine Allylgruppe,
- R²: steht für Wasserstoff oder eine -COOH-Gruppe, wobei die -COOH-Gruppe auch als Salz mit einem physiologisch verträglichen Kation vorliegen kann,
- R³: steht für Wasserstoff, eine C1-C4-Alkylgruppe oder eine Gruppe -CH₂-NR⁷R⁸, in der R⁷ und R⁸ unabhängig voneinander stehen für Wasserstoff oder eine C1-C4-Alkylgruppe,
- R⁴ steht: für Wasserstoff, eine C1-C4-Alkylgruppe oder eine Gruppe -CO-R⁶, in der R⁶ steht für eine C1-C4-Alkylgruppe oder eine gegebenenfalls substituierte Phenylgruppe, und
- R⁵: steht für eine der unter R⁴ genannten Gruppen,
oder ein physiologisch verträgliches Salz dieser Verbindungen mit einer organischen oder anorganischen Säure.

Erfindungsgemäß bevorzugte Vertreter sind das 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol. Der Grundkörper, das 5,6-Dihydroxyindol, ist ganz besonders bevorzugt.

Die in den erfindungsgemäßen Mitteln enthaltenen Indolin- und Indol-Derivate können sowohl als freie Basen als auch in Form ihrer physiologisch verträglichen Salze mit anorganischen oder organischen Säuren, z. B. der Hydrochloride, der Sulfate und Hydrobromide, eingesetzt werden.

Selbstverständlich umfaßt die vorliegende Erfindung auch Mittel, die mehr als ein Indolin- oder Indol-Derivat oder Mischungen von Indolin- und Indol-Derivaten enthalten.

Die Indol- oder Indolin-Derivate sind in den erfindungsgemäßen Mitteln üblicherweise in Mengen von 0,05-10 Gew.-%, vorzugsweise 0,2-5 Gew.-% enthalten.

Im Rahmen dieser Ausführungsform kann bevorzugt sein:
- Hybridfarbstoff der Struktur (I) mit einer Gruppe Y, abgeleitet von einem Oxidationsfarbstoffvorprodukt vom Kupplertyp, in Kombination mit wenigstens einem Derivat des Indols oder Indolins als Vorläufer des Melanins,
- Hybridfarbstoff der Struktur (I) mit einer Gruppe Y, abgeleitet von einem Oxidationsfarbstoffvorprodukt vom Entwicklertyp, in Kombination mit wenigstens einem Derivat des Indols oder Indolins als Vorläufer des Melanins.

Im Rahmen der genannten Ausführungsformen ist es nicht erforderlich, daß die Oxidationsfarbstoffvorprodukte, direktziehenden Farbstoffe oder Melanin-Vorläufer jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Bezüglich der in den erfindungsgemäßen Haarfärbe- und Tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Erfindungsgemäße Mittel, enthaltend einen Hybridfarbstoff der Struktur (I), in der sich die Gruppen X von einem Melaninvorläufer von Indol- oder Indolin-Typ ableiten, enthalten gemäß einer bevorzugten Variante dieser Ausführungsform außer den genannten Hybridfarbstoffen keine weiteren Farbstoffe oder Farbstoffvorprodukte.

Gemäß einer weiteren bevorzugten Variante enthalten insbesondere erfindungsgemäße Mittel mit einem Hybridfarbstoff der Struktur (I), in der sich die Gruppen X von einem Melaninvorläufer von Indol- oder Indolin-Typ ableiten, und/oder mit einem Melanin-Vorläufer weiterhin wenigstens eine Aminosäure oder ein Oligopeptid.

Aminosäuren im Sinne der Erfindung sind solche Substanzen, die wenigstens eine Aminogruppe und wenigstens eine -COOH- oder -SO₃H-Gruppe aufweisen.

Bevorzugte Aminosäuren sind Aminocarbonsäuren, insbesondere α-Aminocarbonsäuren und ω-Aminocarbonsäuren. Unter den α-Aminocarbonsäuren sind wiederum Arginin, Lysin, Ornithin und Histidin besonders bevorzugt.

Die Aminosäuren können den erfindungsgemäßen Mitteln bevorzugt in freier Form zugegeben werden. Es ist auch möglich, die Aminosäuren in Salzform einzusetzen. Bevorzugte Salze sind dann die Verbindungen mit Halogenwasserstoffsäuren, insbesondere die Hydrochloride und die Hydrobromide.

Ganz besonders bevorzugte Aminosäuren sind Lysin und insbesondere Arginin, insbesondere in freier Form, aber auch als Hydrochlorid eingesetzt.

Weiterhin können die Aminosäuren auch in Form von Oligopeptiden und Proteinhydrolysaten eingesetzt werden, wenn sichergestellt ist, daß die erforderlichen Mengen an Verbindungen, die der erfindungsgemäßen Definition der Aminosäuren entsprechen, darin enthalten sind. In diesem Zusammenhang wird auf die Offenbarung der DE-OS 22 15 303 verwiesen, auf die ausdrücklich Bezug genommen wird.

Selbstverständlich umfaßt die Erfindung auch solche Mittel, die zwei und mehr Aminosäuren oder Oligopeptide enthalten. Bevorzugt sind dabei Kombinationen von Arginin mit einer weiteren Aminosäure oder einem Oligopeptid.
Die erfindungsgemäßen Mittel enthalten die Aminosäure bzw. das Oligopeptid bevorzugt in Mengen von 0,1 bis 10 Gew.-%, insbesondere 1 bis 4 Gew.-%, bezogen auf das gesamte Mittel.

Haarfärbemittel, insbesondere wenn die Ausfärbung oxidativ, sei es mit Luftsauerstoff oder anderen Oxidationsmitteln wie Wasserstoffperoxid, erfolgt, werden üblicherweise schwach sauer bis alkalisch, d. h. auf pH-Werte im Bereich von etwa 5 bis 11, eingestellt.
Zu diesem Zweck enthalten die Färbemittel Alkalisierungsmittel, üblicherweise Alkali- oder Erdalkalihydroxide, Ammoniak oder organische Amine.

Gemäß einer speziellen Ausführungsform der vorliegenden Erfindung dient die Aminosäure oder das Oligopeptid nicht nur der Intensivierung der Ausfärbung, sondern übernimmt auch, zumindest teilweise, die Funktion des Alkalisierungsmittels. Im Rahmen dieser Ausführungsform werden daher bevorzugt solche Aminosäuren und Oligopeptide eingesetzt, deren 2,5 Gew.-%ige Lösungen in Wasser einen pH-Wert von 9 und größer aufweisen. Solche Aminosäuren sind die bevorzugt eingesetzten Verbindungen Arginin und Lysin. Im Rahmen dieser Ausführungsform wird das weitere Alkalisierungsmittel bevorzugt ausgewählt aus der Gruppe, die von Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxiden gebildet wird. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist im Rahmen dieser Ausführungsform bevorzugt.

Besonders vorteilhafte Eigenschaften wurden bei Mitteln gefunden, bei denen die Aminosäure oder das Oligopeptid und das weitere Alkalisierungsmittel in einem Verhältnis von 1:5 bis 5:1 vorlagen. Mengenverhältnisse von 1:2 bis 2:1 haben sich als besonders geeignet erwiesen.

Zur Herstellung der erfmdungsgemäßen Färbemittel werden die oben genannten zwingenden und fakultativen Bestandteile in einen geeigneten, bevorzugt wasserhaltigen, Träger eingearbeitet. Solche Träger sind z. B. Cremes, Emulsionen, Gele oder auch tensidhaltige schäumende Lösungen, z. B. Shampoos, Schaumaerosole oder andere Zubereitungen, die für die Anwendung auf dem Haar geeignet sind. Diese Formulierungsformen werden mit den bereits oben erwähnten Alkalisierungsmitteln oder geeigneten Säuren, wie insbesondere Genußsäuren wie Citronensäure, Weinsäure, Milchsäure und Essigsäure, bevorzugt auf einen pH-Wert von 5 bis 11, insbesondere von 7 bis 10, eingestellt.

Weiterhin können die erfindungsgemäßen Färbemittel alle in solchen Zubereitungen bekannten Wirk-, Zusatz- und Hilfsstoffe enthalten. In vielen Fällen enthalten die Färbemittel wenigstens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen. Anionische Tenside können dabei ganz besonders bevorzugt sein.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ether-, Amid- und Hydroxylgruppen sowie in der Regel auch Estergruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare und verzweigte Fettsäuren mit 8 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(-CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylen glykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungs produkte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C8-C22-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül wenigstens eine quartäre Ammoniumgruppe und wenigstens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylamino-propyl-dimethyl-ammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacyl-aminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C8-C18-Alkyl- oder -Acylgruppe im Molekül wenigstens eine freie Aminogruppe und wenigstens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C12-18-Acylsarcosin.

Nichtionische Tenside enthalten als hydrophile Gruppe z. B. eine Polyolgruppe, eine Polyalkylenglykolethergruppe oder eine Kombination aus Polyol- und Polyglykol-ethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C12-C22-Fettsäuremono- und -diester von Anlagerungsprodukten von 1 bis 30 Mol Ethylenoxid an Glycerin,
- C8-C22-Alkylmono- und -oligoglycoside, deren ethoxylierte Analoga und deren Ester z. B. mit Weinsäure und Citronensäure,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester,
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldi-methylammoniumchloride und Trialkylmethylammoniumchloride, z. B. Cetyltrimethyl-ammoniumchlorid, Stearyltrimethylammoniumchlorid, Distearyldimethylammonium-chlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Coming; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquatemäre Polydimethylsiloxane, Quatemium-80).

Alkylamidoamine, insbesondere Fettsäureamidoamine wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex^{®} vertriebenen Methyl-hydroxyalkyl-dialkoyloxyalkyl-ammonium-methosulfate sowie die entsprechenden Produkte, die unter dem Warenzeichen Dehyquart^{®} im Handel erhältlich sind.

Ein Beispiel für ein als kationisches Tensid einsetzbares quaternäres Zuckerderivat stellt das Handelsprodukt Glucquat^{®}100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Erfindungsgemäß kann die Verwendung anionischer Tenside in Kombination mit zwitterionischen Tensiden besonders bevorzugt sein.

Erfindungsgemäß bevorzugt sind ebenfalls solche Mittel, die zusätzlich ein Polymer, ausgewählt aus der Gruppe, die anionische, zwitterionische, ampholytische, kationische und nichtionische Polymere umfaßt, enthalten.
Erfindungsgemäß besonders bevorzugt sind solche Mittel, die zusätzlich ein kationisches Polymer enthalten.

Unter den kationischen Polymeren sind dabei die permanent kationischen Polymere bevorzugt. Als "permanent kationisch" werden erfindungsgemäß solche Polymeren bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten.

Bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning^{®} 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®-}Quat 3270 und 3272 (Hersteller: Th. Goldschmidt; diquaternäre Polydimethylsiloxane, Quaternium-80),
- Kationische Guar-Derivate, wie insbesondere die unter den Handelsnamen Cosmedia^{®}Guar und Jaguar^{®} vertriebenen Produkte,
- Polymere Dimethyldiallylammoniumsalze und deren *Copolymere* mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden.
- quaternierter Polyvinylalkohol,
- sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), Polyquaternium-32, Polyquaternium-35 und Polyquaternium-37 (Handelsprodukte z. B. Salcare^{®} SC 92 und Salcare^{®}SC 95) bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Erfindungsgemäß bevorzugte kationische Polymere sind quaternisierte Cellulose-Derivate, polymere Dimethyldiallylammoniumsalze, Polyquaternium-27 und deren Copolymere sowie Polymere vom Typ Polyquaterniuim-2. Kationische Cellulose-Derivate, insbesondere das Handelsprodukt Polymer^{®}JR 400, und Polymere vom Typ Polyquaternium-2, insbesondere das Handelsprodukt Mirapol^{®}A-15, sind ganz besonders bevorzugte kationische Polymere.

Die kationischen Polymere sind in den erfindungsgemäßen Mitteln bevorzugt in Mengen von 0,05 bis 5 Gew.-%, bezogen auf die gesamte Zubereitung, enthalten.

In vielen Fällen können als Alternative zu den kationischen Polymeren auch Ampho-Polymere eingesetzt werden. Unter dem Oberbegriff Ampho-Polymere sind amphotere Polymere, d. h. Polymere, die im Molekül sowohl freie Aminogruppen als auch freie - COOH- oder SO₃H-Gruppen enthalten und zur Ausbildung innerer Salze befähigt sind, zwitterionische Polymere, die im Molekül quartäre Ammoniumgruppen und -COO-- oder -SO₃⁻-Gruppen enthalten, und solche Polymere zusammengefaßt, die -COOH- oder SO₃H-Gruppen und quartäre Ammoniumgruppen enthalten. Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt. Ebenfalls bevorzugte Amphopolymere setzen sich aus ungesättigten Carbonsäure (z. B. Acryl- und Methacrylsäure), kationisch derivatisierten ungesättigten Carbonsäure (z. B. Acrylamidopropyl-trimethyl-ammoniumchlorid) und gegebenenfalls weiteren ionischen oder nichtionogenen Monomeren zusammen, wie beispielsweise in der deutschen Offenlegungsschrift 39 29 973 und dem dort zitierten Stand der Technik zu entnehmen sind. Ebenfalls erfindungsgemäß einsetzbar sind Terpolymere von Acrylsäure, Methylacrylat und Methacrylamidopropyltrimoniumchlorid, wie sie unter der Bezeichnung Merquat^{®}2001 N im Handel erhältlich sind, sowie das Handelsprodukt Merquat^{®}280.

Ebenfalls bevorzugt enthalten die erfindungsgemäßen Mittel wenigstens ein nichtionogenes oder anionisches Polymer mit verdickenden Eigenschaften. Bevorzugt sind dabei, gegebenenfalls vemetzte, Polyacrylsäuren, Cellulose-Derivate, z. B. Methylcellulose, Hydroxyalkylcellulose und Carboxymethylcellulose, und Xanthan-Gum.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- anionische Polymere wie beispielsweise Polyacrylsäuren, vemetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butylacrylamid-Terpolymere,
- Verdickungsmittel wie Agar-Agar, Guar-Gum, Alginate, Gummi arabicum, Karaya-Gummi, Johannisbrotkernmehl, Leinsamengummen, Dextrane, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose, Maleinsäure und Milchsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin, Diethylenglykol, Ethoxybutanol und Butoxyethanol sowie Benzylalkohol,
- Antischuppenwirkstoffe wie Piroctone Olamine und Zink Omadine,
- weitere Substanzen zur Einstellung des pH-Wertes, wie beispielsweise α- und β-Hydroxycarbonsäuren,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft,
- Antioxidantien.

Die Bestandteile des wasserhaltigen Trägers werden zur Herstellung der erfindungsgemäßen Färbemittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Bezüglich der weiteren Bestandteile der erfindungsgemäßen Färbemittel wird auch ausdrücklich auf die dem Fachmann bekannten Monographien, z. B. Umbach, Kosmetik, 2.

Auflage, Georg Thieme Verlag, Stuttgart New York, 1995, und Kh. Schrader, Grundlagen und Rezepturen der Kosmetika, 2. Auflage, Hüthig Buch Verlag, Heidelberg, 1989, verwiesen.

Zur Ausbildung der Färbung auf der keratinischen Faser können alle bekannten Verfahren angewendet werden.

Gemäß einer bevorzugten Ausführungsform erfolgt die Ausbildung der Färbung mit Luftsauerstoff als einzigem Oxidationsmittel. Diese Ausführungsform ist insbesondere dann bevorzugt, wenn die Gruppe Y des Hybridfarbstoffes sich von luftoxidablen Entwickler- und Kupplerkomponenten ableitet, oder das Mittel Vorläufer des Melanins und/oder luftoxidable Farbstoffvorprodukte vom Kuppler- oder Entwicklertyp enthält. Als luftoxidable Verbindungen sind erfindungsgemäß solche Verbindungen oder Farbstoffvorprodukte zu verstehen, bei denen die oxidative Ausbildung der endgültigen Färbung allein mit Hilfe von Luftsauerstoff ohne Verwendung üblicher chemischer Oxidationsmittel erfolgen kann. Triaminobenzol-Derivate sind Beispiele für solche luftoxidablen Verbindungen.

In bestimmten Fällen, in denen die Gruppe Y des Hybridfarbstoffes sich von Entwickler- und Kupplerkomponenten ableitet, oder das Mittel Vorläufer des Melanin und/oder Farbstoffvorprodukte vom Kuppler- oder Entwicklertyp enthält, kann die Mitverwendung eines chemischen Oxidationsmittels aber gewünscht sein. Dies gilt auch, wenn neben der Färbung ein Aufhelleffekt an menschlichem Haar gewünscht ist. Als Oxidationsmittel kommen dann Persulfate, Chlorite und insbesondere Wasserstoffperoxid oder dessen Anlagerungsprodukte an Harnstoff, Melamin sowie Natriumborat in Frage..

Zweckmäßigerweise wird die Zubereitung des Oxidationsmittels unmittelbar vor dem Färben der Haare mit der Zubereitung mit den Farbstoffvorprodukten vermischt. Das dabei entstehende gebrauchsfertige Haarfärbepräparat sollte bevorzugt einen pH-Wert im Bereich von 5 bis 11 aufweisen. Besonders bevorzugt ist die Anwendung der Haarfärbemittel in einem schwach alkalischen Milieu. Die Anwendungstemperaturen können in einem Bereich zwischen 15 und 40 °C, bevorzugt bei der Temperatur der Kopfhaut, liegen. Nach einer Einwirkungszeit von ca. 5 bis 45, insbesondere 15 bis 30, Minuten wird das Haarfärbemittel durch Ausspülen von dem zu färbenden Haar entfernt. Das Nachwaschen mit einem Shampoo entfällt, wenn ein stark tensidhaltiger Träger, z. B. ein Färbeshampoo, verwendet wurde.

Insbesondere bei schwer färbbarem Haar kann die Zubereitung mit den Farbstoffvorprodukten ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.
Gemäß einer besonderen Ausführungsform dieses Verfahrens erfolgt die Ausbildung der endgültigen Färbung durch mehrmaliges Aufbringen des Mittels und jeweils anschließender Luftoxidation. Das jeweilige Aufbringen des Mittels erfolgt dabei bevorzugt in Abständen, die zwischen etwa einem Tag und etwa zwei Wochen liegen. Dadurch können sehr gezielt spezielle Nuancen erhalten werden.

Unabhängig davon, welches der oben genannten Verfahren zur Anwendung des erfindungsgemäßen Mittels gewählt wird, kann die Ausbildung der Färbung dadurch unterstützt und gesteigert werden, daß dem Mittel bestimmte Metallionen zugesetzt werden. Solche Metallionen sind beispielsweise Zn²⁺, Cu²⁺, Fe²⁺, Fe³⁺, Mn²⁺, Mn⁴⁺, Li⁺, Ru³⁺, Mg²⁺, Ca²⁺ und Al³⁺. Besonders geeignet sind dabei Zn²⁺, Cu²⁺, Mn²⁺, Ru³⁺ und Ca²⁺. Die Metallionen können prinzipiell in der Form eines beliebigen, physiologisch verträglichen Salzes eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflußt werden.

Die oxidative Entwicklung der Färbung kann grundsätzlich mit Luftsauerstoff erfolgen. Bevorzugt wird jedoch, wie zuvor erwähnt wurde, ein chemisches Oxidationsmittel eingesetzt. Man kann aber das Oxidationsfärbemittel auch zusammen mit einem Katalysator auf das Haar aufbringen, der die Oxidation der Farbstoffvorprodukte, z.B. durch Luftsauerstoff, aktiviert. Solche Katalysatoren sind z.B. Übergangsmetallverbindungen, Iodide, Chinone oder bestimmte Enzyme. Geeignete Enzyme sind z.B. Peroxidasen, die die Wirkung geringer Mengen an Wasserstoffperoxid deutlich verstärken können. Ein Beispiel für ein derartiges enzymatisches Verfahren stellt das Vorgehen dar, die Wirkung geringer Mengen (z. B. 1% und weniger, bezogen auf das gesamte Mittel) Wasserstoffperoxid durch Peroxidasen zu verstärken.
Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, *oder in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Ein weiterer Gegenstand der Erfindung ist daher ein Verfahren zum Färben von Keratinfasern, insbesondere von Haaren, bei dem man auf die Faser ein erfindungsgemäßes Oxidationsfärbemittel zusammen mit einem Oxidationsmittel und/oder mit einem Katalysator zur Aktivierung der Oxidation aufbringt und nach einer Einwirkungszeit wieder mit Wasser oder mit einer wäßrigen Tensidzubereitung abspült.

Ein weiterer Gegenstand der Erfindung ist die Verwendung eines Hybridfarbstoffes der Struktur (I) oder einer Mischung dieser Hybridfarbstoffe zum Färben keratinischer Fasern, insbesondere menschlicher Haare.

Weiterhin hat sich gezeigt, daß die erfindungsgemäßen Hybridfarbstoffe sich auch hervorragend zur Anfärbung, insbesondere zur Bräunung, der menschlichen Haut eignen. Ein weiterer Gegenstand der Erfindung ist daher die Verwendung eines Hybridfarbstoffes der Struktur (I) oder einer Mischung dieser Hybridfarbstoffe zum Färben der menschlichen Haut.

Die nachfolgenden Beispiele sollen den Erfindungsgegenstand näher erläutern.

### Beispiele

### 1. Synthesen

### 1.1. 4-(5,6-Dimethoxyindolin-1-yl)phenylamin-hydrochlorid (Hybridfarbstoff A)

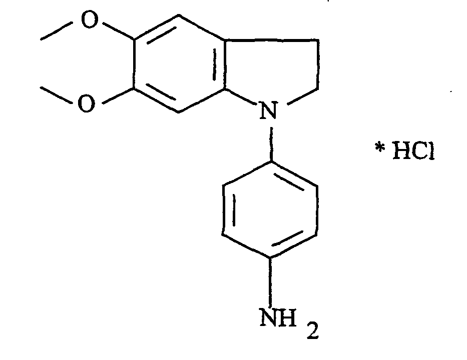

- X: abgeleitet von einem Melanin-Vorläufer (5,6-Dimethoxyindolin)
- Y: abgeleitet von einem Oxidationsfarbstoffvorprodukt vom Entwicklertyp (p-Phenylendiamin)
- S: steht für ein N-Strukturelement (= N des Fünfrings des 5,6-Dimethoxyindolins), das der gemeinsame Bestandteil der Gruppen X und Y ist

### 1. Stufe

Es wurden 6,3 g 4-Nitrofluorbenzol (0,045 mol) mit 8,0 g 5,6-Dimethoxyindolin (0,045 mol) und 4,2 g Natriumhydrogencarbonat (0,050 mol) 2,5 h bei 80 °C in 50 ml DMSO gerührt. Nach dem Abkühlen wurde auf Eis gegeben, der Niederschlag abgesaugt und im Vakuum bei 45 °C getrocknet. Das Zwischenprodukt 5,6-Dimethoxy-1-(4-nitrophenyl)-indolin wurde in einer Ausbeute von 65,6 % und mit einem Schmelzpunkt von 176 - 179 °C erhalten.

### 2. Stufe

5,6-Dimethoxy-1-(4-nitrophenyl)indolin wurde mit Pd (5 %) auf Kohle in Ethanol - Wasser (8:2) in einer Schüttelente über Nacht hydriert. Nach der Beendigung der Wasserstoffaufnahme gab man 10 %ige Salzsäure zu, filtrierte den Katalysator ab und engte zur Trockne ein. Der Rückstand wurde im Vakuum bei 45 °C getrocknet. Das Produkt 4-(5,6-Dimethoxyindolinyl)phenylamin-hydrochlorid wurde in einer Ausbeute von 84,9 % und mit einem Schmelzpunkt von 143 - 145 °C erhalten.

### 1.2 6-(5,6-Dimethoxyindolin-1-yl)(3-pyridyl)amin-hydrochlorid (Hybridfarbstoff B)

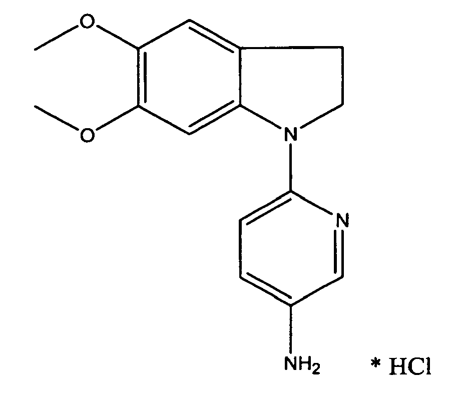

- X: abgeleitet von einem Melanin-Vorläufer (5,6-Dimethoxyindolin)
- Y: abgeleitet von einem Oxidationsfarbstoffvorprodukt vom Entwicklertyp (2,5-Aminopyridin)
- S: steht für ein N-Strukturelement (= N des Fünfrings des 5,6-Dimethoxyindolins), das der gemeinsame Bestandteil der Gruppen X und Y ist

### 1. Stufe

Es wurden 7,1 g 2-Chlor-5-nitropyridin (0,045 mol) mit 8,0 g 5,6-Dimethoxyindolin (0,045 mol), 4,2 g Natriumhydrogencarbonat (0,050 mol) 2,5 h bei 80 °C in 50 ml DMSO gerührt. Nach dem Abkühlen wurde auf Eis gegeben, der Niederschlag abgesaugt und im Vakuum bei 45 °C getrocknet. Das Zwischenprodukt 5,6-Dimethoxy-1-(5-nitro(2-pyridyl)indolin wurde in einer Ausbeute von 23,1 % und mit einem Schmelzpunkt von 202 - 207 °C erhalten.

### 2. Stufe

5,6-Dimethoxy-1-(5-nitro(2-pyridyl))indolin wurden mit Pd (5 %) auf Kohle in Ethanol-Wasser (8:2) in einer Schüttelente über Nacht hydriert. Nach der Beendigung der Wasserstoffaufnahme gab man 10 %ige Salzsäure zu, filtrierte den Katalysator ab und engte zur Trockne ein. Der Rückstand wurde mit Ethanol und Aktivkohle umkristallisiert. Das Produkt 6-(5,6-dimethoxyindolin-1-yl)-3-pyridylamin-hydrochlorid wurde in einer Ausbeute von 70,8 % und mit einem Schmelzpunkt von 168 - 171 °C erhalten.

### 2. Ausfärbungen

Es wurde zunächst eine Cremebasis folgender Zusammensetzung hergestellt [alle Angaben sind, soweit nicht anders vermerkt, in g]:

| | |
|---|---|
| Lorol^{®}techn.¹ | 4,0 |
| Texapon^{®}N 28² | 40,0 |
| Dehyton^{®}K³ | 25,0 |
| Eumulgin^{®}B 2 ⁴ | 1,5 |
| Stenol^{®} 1618⁵ | 17,0 |
| destilliertes Wasser | 12,5 |

| | |
|---|---|
| ¹ C₁₂₋₁₈-Fettalkohol (COGNIS) ² Natriumlaurylethersulfat (ca. 28 % Aktivsubstanz; CTFA-Bezeichnung: Sodium Laureth Sulfate) (COGNIS) ³ Fettsäureamid-Derivat mit Betainstruktur der Formel R-CONH(CH₂)₃N⁺(CH₃)₂CH₂COO⁻ (ca. 30 % Aktivsubstanz; INCI-Bezeichnung Cocoamidopropyl Betaine) (COGNIS) ⁴ Cetylstearylalkohol mit ca. 20 Mol EO (INCI-Bezeichnung: Ceteareth-20) (COGNIS) ⁵ C₁₆₋₁₈-Fettalkohol (INCI-Bezeichnung: Cetearyl Alcohol) (COGNIS) | |

Auf Basis dieser Creme wurde dann folgende Haarfärbecremeemulsion hergestellt:

| | |
|---|---|
| Cremebasis | 50,0 |
| Entwicklerkomponente | 7,5 mmol |
| Kupplerkomponente | 7,5 mmol |
| Na₂SO₃ (Inhibitor) | 1,0 |
| (NH₄)₂SO₄ | 1,0 |
| konz. Ammoniaklösung | ad pH 10 |
| Wasser | ad 100 |

Die Bestandteile wurden der Reihe nach miteinander vermischt. Nach Zugabe der Oxidationsfarbstoffvorprodukte und des Inhibitors wurde zunächst mit konzentrierter Ammoniaklösung der pH-Wert der Emulsion auf 10 eingestellt, dann wurde mit Wasser auf 100 g aufgefüllt.

Die oxidative Entwicklung der Färbung wurde mit 3 %iger Wasserstoffperoxidlösung als Oxidationslösung durchgeführt. Hierzu wurden 100 g der Emulsion mit 50 g Wasserstoffperoxidlösung (3 %ig) versetzt und vermischt.

Die Färbecreme wurde auf ca. 5 cm lange Strähnen standardisierten, zu 90 % ergrauten aber nicht besonders vorbehandelten Menschenhaar aufgetragen und dort 30 Minuten bei 32 °C belassen. Nach Beendigung des Färbeprozesses wurde das Haar gespült, mit einem üblichen Haarwaschmittel gewaschen und anschließend getrocknet.

Als weitere Farbstoffe/Farbstoffvorprodukte wurden folgende Verbindungen eingesetzt: Oxidationsfarbstoffvorprodukte vom Kupplertyp
- K1: Resorcin
- K2: 1-Naphthol
- K3: 3-Aminophenol
- K4: 2,4-Diaminophenoxyethanol · 2 HCl
- K5: 1,3-Bis-(2,4-diaminophenoxy)-propan · 4 HCl · H₂O
- K6: 3-Aminoanilin · 2 HCl

Die Ergebnisse der Ausfärbungen sind in der folgenden Tabelle zusammengestellt:

| **Hybridfarbstoff** | **weiteres Farbstoffvorprodukt** | **Nuance des gefärbten Haares** |
|---|---|---|
| A | K1 | grautürkis |
| A | K2 | türkisblau |
| A | K3 | grautürkis |
| A | K4 | grautürkis |
| A | K5 | olivgrau |
| A | K6 | grautürkis |
| B | K2 | dunkelblau |

## Patentansprüche

1. Indol- und Indolin-Hybridfarbstoffe und -hybridfarbstoffvorprodukte, insbesondere zum Färben keratinischer Fasern, der Struktur (I),
X - S - Y (I)
in der X steht für eine Gruppe, die abgeleitet ist von einem Derivat des Indols oder Indolins als Vorläufer des Melanins;
Y steht für eine Gruppe, die abgeleitet ist von
- einem Oxidationsfarbstoffvorprodukt vom Kuppler- oder Entwicklertyp,
S steht für ein Strukturelement, das ein gemeinsamer Bestandteil der Gruppen X und
Y ist, eine direkte Bindung oder wenigstens eine Spacer-Gruppe.

2. Mittel zum Färben keratinischer Fasern, **dadurch gekennzeichnet, daß** es wenigstens einen Indol- und/oder Indolin-Hybridfarbstoff und/oder -hybridfarbstoffvorprodukt der Struktur (I) enthält,
X - S - Y (I)
in der X steht für eine Gruppe, die abgeleitet ist von einem Derivat des Indols oder Indolins als Vorläufer des Melanins;
Y steht für eine Gruppe, die abgeleitet ist von
- einem Oxidationsfarbstoffvorprodukt vom Kuppler- oder Entwicklertyp,
S steht für ein Strukturelement, das ein gemeinsamer Bestandteil der Gruppen X und
Y ist, eine direkte Bindung oder wenigstens eine Spacer-Gruppe.

3. Mittel nach Anspruch 2, **dadurch gekennzeichnet, daß** es weiterhin ein Oxidationsfarbstoffvorprodukt vom Kuppler- oder Entwicklertyp enthält.

4. Mittel nach Anspruch 2 oder 3, **dadurch gekennzeichnet, daß** es weiterhin einen direktziehenden Farbstoff enthält.

5. Mittel nach einem der Ansprüche 2 bis 4, **dadurch gekennzeichnet, daß** es weiterhin ein Derivat des Indols oder Indolins als Vorläufer des Melanins enthält.

6. Mittel nach einem der Ansprüche 2 bis 5, **dadurch gekennzeichnet, daß** es ein Tensid enthält, ausgewählt aus der Gruppe, umfassend anionische, zwitterionische, ampholytische, kationische und/oder nichtionische Tenside.

7. Mittel nach einem der Ansprüche 2 bis 6, **dadurch gekennzeichnet, daß** es ein Polymer enthält, ausgewählt aus der Gruppe, umfassend kationische, anionische, nichtionogene und/oder Ampho-Polymere.

8. Verwendung eines Hybridfarbstoffes der Struktur (I) nach Anspruch 1 zum Färben keratinischer Fasern, insbesondere menschlicher Haare.

9. Verwendung eines Hybridfarbstoffes der Struktur (I) nach Anspruch 1 zum Färben der menschlichen Haut.

## Claims

1. Indole and indoline hybrid dyes and hybrid dye precursors, more particularly for coloring keratin fibers, corresponding to formula (I):
X - S - Y (I)
where
X is a group derived from an indole or indoline derivative as a melanin precursor,
Y is a group derived from
- an oxidation dye precursor of the secondary or primary intermediate type!
S is a structural element which is common constituent of the groups X and Y, a direct bond or at least one spacer group.

2. A composition for coloring keratin fibers, **characterized in that** it contains at least one indole and/or indoline hybrid dye and/or hybrid dye precursor corresponding to formula (I):
X - S - Y (I)
where
X is a group derived from an indole or indoline derivative as a melanin precursor,
Y is a group derived from
- an oxidation dye precursor of the secondary or primary intermediate type
S is a structural element which is common constituent of the groups X and Y, a direct bond or at least one spacer group.

3. A composition as claimed in claim 2, **characterized in that** it additionally contains an oxidation dye precursor of the secondary or primary intermediate type.

4. A composition as claimed in claim 2 or 3, **characterized in that** it additionally contains a substantive dye.

5. A composition as claimed in any of claims 2 to 4, **characterized in that** it additionally contains a derivative of indole or indoline as the melanin precursor.

6. A composition as claimed in any of claims 2 to 5, **characterized in that** it contains a surfactant selected from the group consisting of anionic, zwitterionic, ampholytic, cationic and/or nonionic surfactants.

7. A composition as claimed in any of claims 2 to 6, **characterized in that** it contains a polymer selected from the group consisting of cationic, anionic, nonionic and/or amphoteric polymers.

8. The use of the hybrid dye of formula (I) claimed in claim 1 for coloring keratin fibers, more particularly human hair.

9. The use of the hybrid dye of formula (I) claimed in claim 1 for coloring human skin.

## Revendications

1. Colorants hybrides et précurseurs de colorants hybrides à base d'indole et d'indoline, en particulier pour la teinture de fibres kératiniques, présentant la structure (I),
X - S - Y (I)
dans laquelle X représente un groupe qui est dérivé d'un dérivé de l'indole ou de l'indoline comme précurseur de la mélanine ;
Y représente un groupe qui est dérivé
- d'un précurseur d'un colorant d'oxydation du type agent de couplage ou de développement,
S représente un élément de structure qui est un constituant commun des groupes X et Y, une liaison directe ou au moins un groupe écarteur.

2. Agent pour la teinture de fibres kératiniques, **caractérisé en ce qu'**il contient au moins un colorant hybride et/ou un précurseur d'un colorant hybride d'indole et/ou d'indoline de structure (I),
X - S - Y (I)
dans laquelle X représente un groupe qui est dérivé d'un dérivé de l'indole ou de l'indoline comme précurseur de la mélanine ;
Y représente un groupe qui est dérivé
- d'un précurseur d'un colorant d'oxydation du type agent de couplage ou de développement,
S représente un élément de structure qui est un constituant commun des groupes X et Y, une liaison directe ou au moins un groupe écarteur.

3. Agent selon la revendication 2, **caractérisé en ce qu'**il contient en outre un précurseur d'un colorant d'oxydation du type agent de couplage ou de développement.

4. Agent selon la revendication 2 ou 3, **caractérisé en ce qu'**il contient en outre un colorant montant directement sur la fibre.

5. Agent selon l'une quelconque des revendications 2 à 4, **caractérisé en ce qu'**il contient en outre un dérivé de l'indole ou de l'indoline comme précurseur de la mélanine.

6. Agent selon l'une quelconque des revendications 2 à 5, **caractérisé en ce qu'**il contient un agent tensioactif, choisi dans le groupe comprenant les agents tensioactifs anioniques, zwittérioniques, ampholytiques, cationiques et/ou non ioniques.

7. Agent selon l'une quelconque des revendications 2 à 6, **caractérisé en ce qu'**il contient un polymère, choisi dans le groupe comprenant les polymères cationiques, anioniques, non ionogènes et/ou amphotères.

8. Utilisation d'un colorant hybride de structure (I) selon la revendication 1 pour la teinture de fibres kératiniques, en particulier des cheveux humains.

9. Utilisation d'un colorant hybride de structure (I) selon la revendication 1 pour la teinture de la peau humaine.
